# EUROPEAN PATENT APPLICATION

(11) **EP 1 099 761 A1**
(43) Date of publication of application: **16.05.2001**
(21) Application number: 99850169.6
(22) Date of filing: 12.11.1999
(51) Int. Cl.: C12N 15/54, C12N 15/82, C12N 15/81, C12N 1/18, C12N 9/10, C11B 1/00

(54) **Use of a class of enzymes to increase the oil content in transgenic organisms**

(71) Applicant: Scandinavian Biotechnology Research AB (ScanBi AB), 268 31 Svalöv (SE)
(72) Inventor: Banas, Antoni, 08-110 Siedlce (PL); Sandager, Line, 2100 Copenhagen (DK); Stahl, Ulf, 753 24 Uppsala (SE); Dahlqvist, Anders, 24466 Furulund (SE); Lenman, Marit, 223 59 Lund (SE); Ronne, Hans, 756 54 Uppsala (SE); Stymne, Sten, 268 90 Svalöv (SE)
(74) Representative: Aldenbäck, Ulla Christina

(57) **Abstract**

The present invention relates to an enzyme, acyl-CoA:diacylglycerol acyltransferase (DAGAT), catalysing the transfer of fatty acids from acyl-CoA to diacylglycerol in the biosynthetic pathway for the production of triacylglycerols. Furthermore, the invention relates to the isolation, identification, characterisation and use of recombinant DNA molecules encoding this class of enzymes.

## Description

### FIELD OF THE INVENTION

The present invention relates to the use of a novel enzyme and its encoding gene for transformation. More specifically, the invention relates to use of a gene encoding an enzyme with acyl-CoA:diacylglycerol acyltransferase activity. This gene, when expressed alone in transgenic organisms, will enhance the total amount of oil *(i.e.* triacylglycerols) produced in the cells.

### BACKGROUND TO THE INVENTION

In oil crops like rape, sunflower, oilpalm etc., the oil (*i.e.* triacylglycerols) is the most valuable product of the seeds or fruits and other compounds like starch, protein and fibre are regarded as by-products with less value. Enhancing the quantity of oil per weight basis at the expense of other compounds in oil crops would therefore increase the value of the crop. If genes regulating the allocation of reduced carbon into the production of oil can be overexpressed, the cells will accumulate more oil at the expense of other products. Such genes might not only be used in already high oil producing organisms such as in oil crops, they could also lead to significant oil production in moderate or low oil containing crops such as e.g. soy, oat, maize, potato, sugar beats, and turnips as well as in microorganisms.

Developments in genetic engineering technologies combined with a greater understanding of the biosynthesis of triacylglycerols now make it possible to transfer genes coding for key enzymes involved in the synthesis of triacylglycerols from a wild species or organisms of other kingdoms into domesticated oilseed crops. In this way triacylglycerols can be produced in high purity and quantities at moderate costs.

### PRIOR ART

Within prior art it is known that the biosynthesis of triacylglycerols (TAG) in fataccumulating tissues in animals (Bell, R.M. & Coleman, R.A., 1983) and plants (Cao, Y.-Z. & Huang A.H.C., 1986, Martin, B.A. & Wilson, R.F., 1983) as well as the accumulation of oil in microbial organisms such as bacteria (Ekundayo R. & Packter, N.M., 1994), yeast and other fungi (Ratledge, C. 1989) are catalysed by acyl-CoA:diacylglycerol acyltransferases (DAGAT), enzymes that transfer an acyl-group from acyl-CoA to diacylglycerol, thus forming TAG.

Furthermore, prior art describes the identification of genes encoding a DAGAT in mouse (Cases et al., 1998) and plants (Hobbs et al., 1999). These genes shares regions of homology with genes encoding acyl-CoA:cholesterol acyltransferases (ACAT).

Prior art also describes the identification of two ACAT encoding genes, *ARE1* and *ARE2,* in the baker's yeast *Saccharomyces cerevisiae* (Yang et al., 1989), both of which also showed significant homology to the DAGAT gene from mouse. Mutants disrupted in these genes were reported not to show any significant changes in the TAG accumulation as judged by metabolic and ultra-structural studies (Yang et al., 1989).

### SUMMARY OF INVENTION

The major route for the synthesis of triacylglycerol (TAG) in yeast as well in all TAG accumulating organisms has been suggested to occur via the acylation of diacylglycerol (DAG) catalysed by acyl-CoA:diacylglycerol acyltransferase (DAGAT). Genes encoding such enzymes has recently been identified (Cases, et al., 1998, Hobbs et al., 1999) and were shown to be homologous to acyl-CoA: cholesterol acyltransferases (ACAT).

A yeast mutant, disrupted in the *ARE1* gene which is homologous to the animal DAGAT genes, was obtained, and the TAG accumulation in the mutant was shown to be significantly lowered at late growth stages, as compared to a congenic wild type yeast. It was also shown that over-expression of the *ARE1* gene in yeast increases the total amount of TAG accumulated in the cells. Furthemore, microsomal membranes prepared from yeast overexpressing the *ARE1* gene were shown to have an increased DAGAT activity, as compared to the control yeast. This shows that the *ARE1* gene encodes an enzyme with DAGAT activity, and that it can be used to increase the oil content in a cell when over-expressed.

### DETAILED DESCRIPTION OF THE INVENTION

Membrane lipids and triacylglycerols have common biosynthetic pathways (Kennedy, 1961). A single acyl-CoA:diacylglycerol acyltransferase was long considered to be the only enzyme in triacylglycerol synthesis that is able to divert diacylglycerols from membrane lipid synthesis into triacylglycerols (Stymne and Stobart 1987). However, recent work has shown that triacylglycerol synthesis also can occur in a reaction that is catalyzed by two non-homologous DAGAT enzymes (Hobbs et al., 1999 and Lardizabal, 1999).

This invention describes the identification of a gene encoding an enzyme with DAGAT activity that is partly responsible for TAG accumulation in yeast and which differs from the hitherto reported DAGAT genes. Furthermore, it is shown that overexpression of this gene in yeast correlates with increased levels of TAG.

The invention will be described more closely below in association with an experimental part and the enclosed drawings and tables.

### BRIEF DESCRIPTION OF THE DRAWINGS:

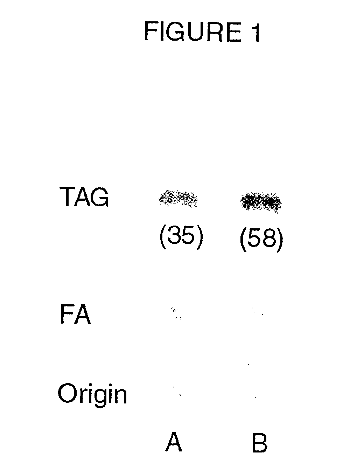

**Figure 1** presents the DAGAT activity in microsomal membranes prepared from a wild type yeast (SCY62) overexpressing the ARE1 gene and a control yeast with an empty plasmid. Aliquots of microsomal membranes prepared from the control yeast (lane A) or the ARE1 overexpressor (lane B) were assayed for DAGAT activity. The reaction mixture was prepared by emulsifying 1 umol of dioleoyl-PG and 0.25 umol of dioleoyl-DAG in 50 ul buffer consisting of 190 mM Hepes-NaOH pH 7.5, 125 mM MgCl₂, 30 mM CHAPS, 2.5 mg/ml BSA and 2 nmol [¹⁴C]-palmitoyl-CoA (2775 dpm/nmol) to which 50 ul of microsomal membranes (10 nmol phosphatidylcholine) was added. The reaction mixture was incubated at 30°C for 30 min. The lipids were then extracted in chloroform and separated by thin layer chromatography on silica gel 60 plates in hexan/dietylether/acetic acid (80:20:1). The radioactive triacylglycerol synthesised was visualised and quantified as cpm (figures in brackets) on the plate by electronic autoradiography (Instant Imager, Packard, US). Abbreviations used in the figure: triacylglycerol (TAG) and unesterified fatty acids (FA).

### BRIEF DESCRIPTION OF THE SEQ ID:

SEQ ID NO. 1: Genomic DNA sequence of the *Saccharomyces cerevisiae ARE1* gene, ORF YCR048W.

SEQ ID NO. 2: The amino acid sequence of the open reading frame YCR048W from *Saccharomyces cerevisiae.*

The present invention is further described by reference to the following non-limiting Examples.

### EXAMPLE 1.

### Triacylglycerol accumulation is reduced in yeast cells that lack the ARE1 gene.

The oil accumulation in yeast cells lacking the *ARE1* gene was analysed and compared to wild type yeast cells at different stages of growth. The yeast cells were harvested in exponential phase after 10 hours of cultivation at 28 °C on a rotary shaker (200 rpm) in YPD medium (2% bacteriological peptone, 1% yeast extract, 2% glucose). The lipid content of the yeast was analysed by TLC and GC. The total amount of lipid accumulated in the *are1* mutant, measured as nmol FA per dry weight yeast, was not significantly different from the wild type yeast (table1) and the amount of fatty acids accumulated into TAG did not differ strongly between the wild-type and the *are1* mutant. The effect of the *are1* disruption on oil accumulation in stationary phase cells was analysed in an experiment were the yeast cells were pre-cultivated for 24 h in liquid YPD medium at 28 °C. The cells were then harvested and re-suspended in minimal medium (Meesters et al, 1996), supplemented with 16 g/l glycerol, to the original volume of the growth culture. In this glycerol supplemented minimal medium the yeast cells will enter stationary phase under conditions suitable for TAG accumulation. After further cultivation for 24 h, the cells were harvested and their lipid composition was determined. The total lipid content in the *are1* mutant was 15% less than in the wild type. The TAG amount in the *are1* mutant was almost 40 % lower than in the wild type, whereas the polar lipid content did not differ significantly between the arel mutant and the wild type yeast (table 1).
In summary, these experiment clearly show that the product of the *ARE1* gene contributes to TAG accumulation in yeast cells under growth conditions that are suitable for TAG accumulation.

**Table 1. *Lipid content of yeast cells disrupted for the ARE1 gene.*** The lipid accumulation in yeast disrupted for the *ARE1* gene (*are1*-mutant) was analysed at different stages of growth and compared to the control wild type yeast. The lipid composition of cells in exponential growth was analysed after 10 hours of cultivation in YPD medium at 28 °C. Yeast cells in stationary phase was prepared by pre-cultivating the cells on liquid YPD medium for 24 hours at 28 °C, after which the cells were harvested, re-suspended in minimal medium (Meesters et al, 1996) supplemented with 16 g/1 glycerol, and cultivated for an additional 24 hours at 28 °C. Lipids were extracted in chloroform, fractionated on TLC and quantified by GC analyses. The lipid content was measured as nmol fatty acids (FA) per mg yeast (dry weight).

| | **Wild type yeast** | | ***are1*-mutant** | |
|---|---|---|---|---|
| | (nmol FA/mg) | | (nmol FA/mg) | |
| | **10h** | **48h** | **10h** | **48h** |
| **Sterol esters** | 15 | 24 | 12 | 19 |
| **Triacylglycerol** | 6 | 44 | 8 | 28 |
| **Other neutral lipids** | 4 | 6 | 4 | 5 |
| **Polar lipids** | 65 | 74 | 63 | 74 |
| **Total lipids** | 90 | 148 | 87 | 126 |

### EXAMPLE 2.

### Triacylglycerol accumulation is increased in yeast cells that overexpress the ARE1 gene.

The effect of over-expression of the *ARE1* gene on lipid accumulation was studied by transforming the wild-type yeast (strain SCY62) with a plasmid containing the *ARE1* gene under control of the galactose-induced *GAL1* promotor (Table 2). Overexpression of the *ARE1* gene from this promoter had no strong effect on the growth rate as determined by optical density measurements. However, the total lipid content in yeast cells that overexpressed *ARE1* was 1.4 fold higher than in the control yeast transformed with an empty expression vector (Table 2). The elevated lipid content in yeast cells overexpressing *ARE1* is mostly due to a 50% increase in the TAG content, but the amount of sterol esters also increased significantly in these cells, as compared to the control. These results clearly demonstrate that the gene product *of ARE1,* in addition to its earlier reported involvement in the synthesis of sterol esters (Yang et al., 1996), also is involved in TAG synthesis. The elevated levels of TAG achieved in the *ARE1* overexpressing cells also clearly demonstrate the potential use of the *ARE1* gene in increasing the oil content in transgenic organisms.

**Table 2. *Lipid content in yeast that overexpress the ARE1 gene.*** Yeast cells transformed with the *ARE1* gene under the control of the *GAL1* promotor in the pJN92 vector were grown in YNB medium at 28 °C. After 43 hours of growth, overexpression *of ARE1* was induced by the addition of 2 % (v/v) galactose. Cells were harvested after an additional 24 hours of growth and analysed for their lipid content. Yeast cells transformed with an empty vector grown under identical conditions was used as control. Lipids were extracted in chloroform, fractionated on TLC and quantified by GC analyses. The lipid content was measured as nmol fatty acids (FA) per mg yeast (dry weight).

| | **control yeast** (nmol FA/mg) | ***ARE1*-overexpressor** (nmol FA/mg) |
|---|---|---|
| **Sterol esters** | 19 | 27 |
| **Triacylglycerol** | 160 | 239 |
| **Other neutral lipids** | 30 | 32 |
| **Polar lipids** | 48 | 56 |
| **Total lipids** | 257 | 354 |

### EXAMPLE 3.

### Diacylglycerol acyltransferase activity is increased in yeast cells overexpressing the ARE1 gene.

A wild type yeast (strain SCY62) was transformed with a plasmid (pJN92) containing the ARE1 gene under the control of a GAL1 promotor. The transformed yeast was cultivated at 28°C in defined YNB medium lacking uracil. The expression of the ARE1 gene was induced by the addition of 2 % (v/v) galactose after 8 hours growth and the cells were harvested after additional 17 hours. Microsomal membranes were prepared from the transformed yeast by resuspending 1g of yeast (fresh weight) with 8 ml of ice-cold buffer (20 mM Tris-Cl, pH 7.9, 10 mM MgCl₂, 1 mM EDTA, 5 % (v/v) glycerol, 1 mM DTT, 0.3 M ammonium sulphate) in a 12 ml glass tube to which 4 ml of glass beads (diameter 0.45 - 0.5 mm) were added. The glass tube was heavily shaken (3 x 60 s) with a MSK cell homogeniser (B. Braun Melsungen AG, Germany). The suspension was centrifuged at 20 000 g for 15 min and the resulting supernatant was centrifuged at 100 000g for 2 h at 6 °C. The resulting pellet, containing microsomal membranes, was resuspended in 0.1 M K-phosphate (pH 7.2) buffer and stored at -80 °C. Microsomal membranes prepared from the transformed yeast overexpressing the ARE1 gene and from a control yeast transformed with an empty plasmid (pJN92) were then assayed for DAGAT activity. The reaction mixture was prepared by emulsifying 1 umol of dioleoyl-PG and 0.25 umol of dioleoyl-DAG in 50 ul of buffer containing 190 mM Hepes-NaOH pH 7.5, 125 mM MgCl₂, 30 mM CHAPS, 2.5 mg/ml BSA and 2 nmol [¹⁴C]-palmitoyl- CoA (2775 dpm/nmol), to which 50 ul of microsomal membranes (10 nmol PC) were added. The reaction mixture was incubated at 30°C for 30 min. The lipids were then extracted in chloroform and separated using thin layer chromatography on silica gel 60 plates in hexan/dietylether/acetic acid (80:20:1). The radioactive lipids were visualised and quantified on the plates by electronic autoradiography (Instant Imager, Packard, US).
The amount of radiolabelled TAG synthesised from [¹⁴C]-palmitoyl-CoA in microsomal membranes prepared from the ARE1 overexpressor was increased with 66 % as compared to the control yeast (Fig 1). Hence, the higher levels of TAG observed in the ARE1-overexpressor (Table 2) can be explained by an increased diacylglycerol acyltransferase activity. These results clearly demonstrate the potential use of the ARE1 gene in increasing the oil content in transgenic organisms.

SEQUENCE LISTING

<110> Scandinavian Biotechnology Research (ScanBi AB) AB, ORG. NO. 556577-
8445

<120> USE OF A CLASS OF ENZYMES AND THEIR ENCODING GENES TO INCREASE THE
OIL CONTENT IN TRANSGENIC ORGANISMS

<160> 2

<210> 1

<211> 1833

<212> DNA

<213> Saccharomyces cerevisiae

<400> 1

atgacggaga ctaaggattt gttgcaagac gaagagtttc ttaagatccg cagactcaat 60
tccgcagaag ccaacaaacg gcattcggtc acgtacgata acgtgatcct gccacaggag 120
tccatggagg tttcgccacg gtcgtctacc acgtcgctgg tggagccagt ggagtcgact 180
gaaggagtgg agtcgactga ggcggaacgt gtggcaggga agcaggagca ggaggaggag 240
taccctgtgg acgcccacat gcaaaagtac ctttcacacc tgaagagcaa gtctcggtcg 300
aggttccacc gaaaggatgc tagcaagtat gtgtcgtttt ttggggacgt gagttttgat 360
cctcgcccca cgctcctgga cagcgccatc aacgtgccct tccagacgac tttcaaaggt 420
ccggtgctgg agaaacagct caaaaattta cagttgacaa agaccaagac caaggccacg 480
gtgaagacta cggtgaagac tacggagaaa acggacaagg cagatgcccc cccaggagaa 540
aaactggagt cgaacttttc agggatctac gtgttcgcat ggatgttctt gggctggata 600
gccatcaggt gctgcacaga ttactatgcg tcgtacggca gtgcatggaa taagctggaa 660
atcgtgcagt acatgacaac ggacttgttc acgatcgcaa tgttggactt ggcaatgttc 720
ctgtgcactt tcttcgtggt tttcgtgcac tggctggtga aaaagcggat catcaactgg 780
aagtggactg ggttcgttgc agtgagcatc ttcgagttgg ctttcatccc cgtgacgttc 840
cccatttacg tctactactt tgatttcaac tgggtcacga gaatcttcct gttcctgcac 900
tccgtggtgt ttgttatgaa gagccactcg tttgcctttt acaacgggta tctttgggac 960
ataaagcagg aactcgagta ctcttccaaa cagttgcaaa aatacaagga atctttgtcc 1020
ccagagaccc gcgagattct gcaaaaaagt tgcgactttt gccttttcga attgaactac 1080
cagaccaagg ataacgactt ccccaacaac atcagttgca gcaatttctt catgttctgt 1140
ttgttccccg tcctcgtgta ccagatcaac tacccaagaa cgtcgcgcat cagatggagg 1200
tatgtgttgg agaaggtgtg cgccatcatt ggcaccatct tcctcatgat ggtcacggca 1260
cagttcttca tgcacccggt ggccatgcgc tgtatccagt tccacaacac gcccaccttc 1320
ggcggctgga tccccgccac gcaagagtgg ttccacctgc tcttcgacat gattccgggc 1380
ttcactgttc tgtacatgct cacgttttac atgatatggg acgctttatt gaattgcgtg 1440
gcggagttga ccaggtttgc ggacagatat ttctacggcg actggtggaa ttgcgtttcg 1500
tttgaagagt ttagcagaat ctggaacgtc cccgttcaca aatttttact aagacacgtg 1560
taccacagct ccatgggcgc attgcatttg agcaagagcc aagctacatt atttactttt 1620
ttcttgagtg ccgtgttcca cgaaatggcc atgttcgcca ttttcagaag ggttagagga 1680
tatctgttca tgttccaact gtcgcagttt gtgtggactg ctttgagcaa caccaagttt 1740
ctacgggcaa gaccgcagtt gtccaacgtt gtcttttcgt ttggtgtctg ttcagggccc 1800
agtatcatta tgacgttgta cctgacctta tga 1833

<210> 2
<211> 610
<212> PRT
<213> Saccharomyces cerevisiae

<400> 2
Met Thr Glu Thr Lys Asp Leu Leu Gln Asp Glu Glu Phe Leu Lys Ile
1               5                   10                  15

Arg Arg Leu Asn Ser Ala Glu Ala Asn Lys Arg His Ser Val Thr Tyr
            20                  25                  30

Asp Asn Val Ile Leu Pro Gln Glu Ser Met Glu Val Ser Pro Arg Ser
        35                  40                  45

Ser Thr Thr Ser Leu Val Glu Pro Val Glu Ser Thr Glu Gly Val Glu
    50                  55                  60

Ser Thr Glu Ala Glu Arg Val Ala Gly Lys Gln Glu Gln Glu Glu Glu
65                  70                  75                  80

Tyr Pro Val Asp Ala His Met Gln Lys Tyr Leu Ser His Leu Lys Ser
                85                  90                  95

Lys Ser Arg Ser Arg Phe His Arg Lys Asp Ala Ser Lys Tyr Val Ser
            100                 105                 110

Phe Phe Gly Asp Val Ser Phe Asp Pro Arg Pro Thr Leu Leu Asp Ser
        115                 120                 125

Ala Ile Asn Val Pro Phe Gln Thr Thr Phe Lys Gly Pro Val Leu Glu
    130                 135                 140

Lys Gln Leu Lys Asn Leu Gln Leu Thr Lys Thr Lys Thr Lys Ala Thr
145                 150                 155                 160

Val Lys Thr Thr Val Lys Thr Thr Glu Lys Thr Asp Lys Ala Asp Ala
                165                 170                 175

Pro Pro Gly Glu Lys Leu Glu Ser Asn Phe Ser Gly Ile Tyr Val Phe
            180                 185                 190

Ala Trp Met Phe Leu Gly Trp Ile Ala Ile Arg Cys Cys Thr Asp Tyr
        195                 200                 205

Tyr Ala Ser Tyr Gly Ser Ala Trp Asn Lys Leu Glu Ile Val Gln Tyr
    210                 215                 220

Met Thr Thr Asp Leu Phe Thr Ile Ala Met Leu Asp Leu Ala Met Phe
225                 230                 235                 240

Leu Cys Thr Phe Phe Val Val Phe Val His Trp Leu Val Lys Lys Arg
                245                 250                 255

Ile Ile Asn Trp Lys Trp Thr Gly Phe Val Ala Val Ser Ile Phe Glu
            260                 265                 270

Leu Ala Phe Ile Pro Val Thr Phe Pro Ile Tyr Val Tyr Tyr Phe Asp
        275                 280                 285

Phe Asn Trp Val Thr Arg Ile Phe Leu Phe Leu His Ser Val Val Phe
    290                 295                 300

Val Met Lys Ser His Ser Phe Ala Phe Tyr Asn Gly Tyr Leu Trp Asp
305                 310                 315                 320

Ile Lys Gln Glu Leu Glu Tyr Ser Ser Lys Gln Leu Gln Lys Tyr Lys
                325                 330                 335

Glu Ser Leu Ser Pro Glu Thr Arg Glu Ile Leu Gln Lys Ser Cys Asp
            340                 345                 350

Phe Cys Leu Phe Glu Leu Asn Tyr Gln Thr Lys Asp Asn Asp Phe Pro
        355                 360                 365

Asn Asn Ile Ser Cys Ser Asn Phe Phe Met Phe Cys Leu Phe Pro Val
    370                 375                 380

Leu Val Tyr Gln Ile Asn Tyr Pro Arg Thr Ser Arg Ile Arg Trp Arg
385                 390                 395                 400

Tyr Val Leu Glu Lys Val Cys Ala Ile Ile Gly Thr Ile Phe Leu Met
                405                 410                 415

Met Val Thr Ala Gln Phe Phe Met His Pro Val Ala Met Arg Cys Ile
            420                 425                 430

Gln Phe His Asn Thr Pro Thr Phe Gly Gly Trp Ile Pro Ala Thr Gln
        435                 440                 445

Glu Trp Phe His Leu Leu Phe Asp Met Ile Pro Gly Phe Thr Val Leu
    450                 455                 460

Tyr Met Leu Thr Phe Tyr Met Ile Trp Asp Ala Leu Leu Asn Cys Val
465                 470                 475                 480

Ala Glu Leu Thr Arg Phe Ala Asp Arg Tyr Phe Tyr Gly Asp Trp Trp
                485                 490                 495

Asn Cys Val Ser Phe Glu Glu Phe Ser Arg Ile Trp Asn Val Pro Val
            500                 505                 510

His Lys Phe Leu Leu Arg His Val Tyr His Ser Ser Met Gly Ala Leu
        515                 520                 525

His Leu Ser Lys Ser Gln Ala Thr Leu Phe Thr Phe Phe Leu Ser Ala
    530                 535                 540

Val Phe His Glu Met Ala Met Phe Ala Ile Phe Arg Arg Val Arg Gly
545                 550                 555                 560

Tyr Leu Phe Met Phe Gln Leu Ser Gln Phe Val Trp Thr Ala Leu Ser
                565                 570                 575

Asn Thr Lys Phe Leu Arg Ala Arg Pro Gln Leu Ser Asn Val Val Phe
            580                 585                 590

Ser Phe Gly Val Cys Ser Gly Pro Ser Ile Ile Met Thr Leu Tyr Leu
        595                 600                 605

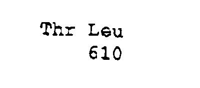

### REFERENCES

Bell, R.M., and Coleman, R.A. (1983) in *The Enzymes* (Boyer, P.D., ed.) Vol. 16, pp. 87-111, Academic Press, Orlando
Cao, Y.-Z. and Huang, A.H.C. (1986) *Plant. Physiol.* **82**, 813-820
Cases, S., Smith, S.J., Zheng, Y.-W., Myers, H.M., Lear, S.R., Sande, E., Novak, S., Collins, C., Welch, C.B., Lusis, A.J., Erickson, S.K. and Farese, R.V. (1998) *Proc. Natl. Acad. Sci.* **95**, 13018-13023
Ekundayo, R.O., and Packter, N.M. (1994) *Microbiology* **140**, 931-943
Hobbs, D.H., Lu, C.F., Hills, M.J., (1999) *FEBS Letters* **452**, 145-149
Kennedy, E.P. (1961) *Fed. Proc. Fed. Am. Soc. Exp. Biol.* **20**, 934-940
Lardizabal, K., Hawkins, D., Mai, J., and Wagner, N. (1999) *Identification of a new diacylglycerol acyltransferase gene family.* Presented at Biochem, Mol. Biol. Plant Fatty Acids Glycerolipids Symp., South Lake Tahoe, Calif., USA.
Martin, B.A. and Wilson, R.F. (1983) *Lipids* **18**, 1-6
Meesters, P.A.E.P., Huijberts, G.N.M. and Eggink, G. (1996) *Appl. Microbiol. Biotechnol.* **45**, 575-579
Ratledge, C. (1989) in *Microbial Lipids* (Ratledge, C. and Wilkinson, S.G., eds.) Vol. 2, pp.567-668, Academic Press, London
Yang, H., Bard, M., Bruner, D.A., Gleeson, A., Deckelbaum, R.J., Aljinovic, G., Pohl, T.M., Rothstein, R. And Sturley, S.L. (1996) **272**, 1353-1356

## Claims

1. A recombinant DNA molecule encoding an enzyme catalysing the transfer of a fatty acid from acyl-CoA to diacylglycerol, resulting in the formation of TAG, wherein said enzyme comprises an amino acid sequence set forth in SEQ ID NO. 2, or fragments thereof.

2. A recombinant DNA molecule encoding a polypeptide with an amino acid sequence that is 60 % or more identical to the sequence set forth in SEQ ID NO. 2.

3. Use of a recombinant DNA molecule, according to claim 1 and 2, for transformation.

4. Use according to claim 3, wherein said DNA molecule is used for transformation of any organism in order to be expressed in this organism and result in an active recombinant enzyme.

5. Use according to claim 4, wherein the transgenic organism is characterised by increased or altered oil content.

6. Transgenic organisms comprising in their genome a DNA molecule, according to claim 1 and 2, transferred by recombinant DNA technology.

7. Transgenic organisms according to claim 6, which are selected from the group consisting of fungi, plants and animals.

8. Transgenic organisms according to claim 6, which are selected from the group consisting of agricultural plants.

9. Transgenic organisms according to claim 6, which are selected from the group consisting agricultural plants and where the said DNA molecule is expressed under the control of a storage organ specific promotor.

10. Transgenic organisms according to claim 6, which are selected from the group consisting agricultural plants and where the said DNA molecule is expressed under the control of a seed specific promotor.

11. Transgenic organisms according to claim 6 to 10 characterised by an increased or altered oil content.

12. Oils from organisms according to claim 6 to 11.
